# EUROPEAN PATENT APPLICATION

(11) **EP 4 537 834 A1**
(43) Date of publication of application: **16.04.2025**
(21) Application number: 23203022.1
(22) Date of filing: 11.10.2023
(51) Int. Cl.: A61K 31/713, A61P 37/06, C12N 15/113, A61K 31/7105

(54) **PRECISION IMMUNOSUPPRESSION AS PREVENTION AND THERAPY FOR GRAFT-VERSUS-HOST DISEASE**

(71) Applicant: Eberhard Karls Universität Tübingen (Medizinische Fakultät), 72074 Tübingen (DE)
(72) Inventor: Haraszti, Reka, 72074 Tübingen (DE); Kremer, Anastasia, 72074 Tübingen (DE)
(74) Representative: Witte, Weller & Partner Patentanwälte mbB

(57) **Abstract**

The present invention relates to a composition, preferably a pharmaceutical composition for the prophylaxis and/or treatment of graft-versus-host disease (GvHD), a combination of inhibitory agents for use in the prophylaxis and/or treatment of GvHD, a stem cell comprising said composition or inhibitory agents, a method of producing said pharmaceutical composition, and to a method of prophylactical and/or therapeutical treatment of a subject suspected of having or developing GvHD.

## Description

The present invention relates to a composition, preferably a pharmaceutical composition for the prophylaxis and/or treatment of graft-versus-host disease (GvHD), a combination of inhibitory agents for use in the prophylaxis and/or treatment of GvHD, a stem cell and lymphocyte composition comprising said composition or inhibitory agents, a method of producing said pharmaceutical composition, and to a method of prophylactical and/or therapeutical treatment of a subject suspected of having or developing GvHD.

### FIELD OF THE INVENTION

The invention relates to the field of molecular immunology.

### BACKGROUND OF THE INVENTION

Graft-versus-host disease (GvHD) is a condition resulting from bone marrow donation or blood stem cell transplant. In particular, GvHD is a serious side effect of allogeneic stem cell transplantation. Allogeneic T cells are effectors of hematopoietic stem cell transplantation (HSCT), delivering potent anti-tumor (GvT) effects, providing anti-infection (Gvl) effects but also occasionally leading to the severe-to-mortal side effect of GvHD. It occurs when the immune cells of the donor reject their new environment and begin to attack the healthy host tissue. Allogeneic stem cell transplantation, in turn, is the only curative option for many leukemias and lymphomas as well as nonmalignant hemate-logic diseases, such as aplastic anemia.

GvHD has a mortality of up to 50%. The remaining patients can be severely limited in their quality of life by GvHD. Current drugs used to treat GvHD also interfere with the graft-versus-leukemia (GvL) and graft-versus-infection (Gvl) effects desired by allogeneic stem cell transplantation. They impair the allogeneic T cells and thus lead to recurrence of the underlying disease and serious infections as well as sepsis. This is associated with a very poor prognosis.

The reason for no existing selective treatment of GvHD is, that no intracellular biological pathway is known that would be selectively responsible for GvHD an not for GvT and Gvl.

Against this background, a precision therapy is urgently needed that would inhibit only GvH in T cells, would not affect GvL and Gvl function of T cells, and, if possible, would not otherwise inhibit other cells in their activity and function.

The task underlying the invention is therefore to provide an active agent and a composition with which the disadvantages of current medicaments against GvHD are avoided or at least reduced. In particular, it is intended to provide such a composition with which selective disease-causing properties of the allogeneic T cells are eliminated or reduced, but the GvL and Gvl functions are at least largely preserved.

### SUMMARY OF THE INVENTION

The problem underlying the invention is solved by a composition comprising an inhibitor of RAs-related nuclear protein (RAN), especially by a pharmaceutical composition comprising said inhibitor, for the prophylaxis and/or treatment of graft-versus-host disease (GvHD).

The inventors have surprisingly realized by screening a large number of potential targets that by the inhibition of the RAs-related nuclear protein (RAN) a selective and specific prophylaxis and treatment of GvHD is possible without negatively affecting graft-versus-leukemia (GvL) and graft-versus-infection (Gvl).

Thereby it is of particular advantage that the effect observed by the inventors is synergistic, i.e., the effect resulting from the combination of both inhibitors is stronger than the sum of the effects of the individual inhibitors.

The findings were surprising and not to be expected.

Ran (RAs-related Nuclear protein) also known as GTP-binding nuclear protein Ran is a protein that in humans is encoded by the RAN gene (Entrez: 5901). Ran is a small 25 kDa protein that is involved in transport into and out of the cell nucleus during interphase and also involved in mitosis. It is a member of the Ras superfamily. Ran is a small G protein that is essential for the translocation of RNA and proteins through the nuclear pore complex. The Ran protein has also been implicated in the control of DNA synthesis and cell cycle progression, as mutations in Ran have been found to disrupt DNA synthesis.

An inhibition of RAN to prevent or counteract the development of GvHD has not yet been described.

According to the invention, an "inhibitor" is a compound or molecule that finally reduces the activity of the target. This inhibition can basically take place in any way, e.g. by reducing the expression (such as transcription, translation, synthesis), reducing the stability or the amount of the target or gene, gene transcript or gene product, or other activity reduction. The inhibitor can therefore both inhibit the coding molecule so that no or less coding product, i.e. protein, is formed. However, the inhibitor can also inhibit the coding product, i.e. protein, itself.

Therefore, the inhibitor can be used in any form, e.g., as a chemically defined substance, such as a small molecule, as an inhibitory nucleic acid molecule, as a protein or peptide, etc.

In an embodiment of the invention the composition additionally comprises an inhibitor of Aurora kinase A (AURKA).

This measure has the advantage of significantly and in a synergistic way increasing or enhancing the selective and specific activity of the composition for the prevention and/or treatment of graft-versus-host disease (GvHD). The inventors were able to demonstrate this increase in efficacy in established experimental systems. Significantly, there is no negative interference with graft-versus-leukemia (GvL) and graft-versus-infection (Gvl) effects of a transplant.

Again, this finding was surprising and could not be expected.

Aurora kinase A also known as serine/threonine-protein kinase 6 is an enzyme that in humans is encoded by the AURKA gene (Entrez: 6790). Aurora A is a member of a family of mitotic serine/threonine kinases. It is implicated with important processes during mitosis and meiosis whose proper function is integral for healthy cell proliferation. So far, Aurora A dysregulation has been associated with high occurrence of cancer. Aurora A has also been shown to be involved in the Epithelial-mesenchymal transition and neuroendocrine transdifferentiation of prostate cancer cells in aggressive disease.

It is mentioned that combined inhibition of AURKA and Janus kinase 2 (JAK2) can prevent xenogeneic GvHD; see Betts et al. (2017), Targeting Aurora kinase A and JAK2 prevents GVHD while maintaining Treg and antitumor CTL function, Sci. Transl. Med. 9(372): eaai8269.

However, a combined inhibition of AURKA or RAN to prevent or counteract the development of GvHD has not yet been described.

In one embodiment of the invention, the composition comprises a further compound, namely an inhibitor of wing apart-like protein homolog (WAPL) and/or an inhibitor of kinesin family member 15 (KIF15).

Again, this measure has the advantage of significantly and in a synergistic way increasing or enhancing the selective and specific activity of the composition for the prevention and/or treatment of graft-versus-host disease (GvHD). The inventors were able to demonstrate this increase in efficacy again in established experimental systems. Significantly, there is no negative interference with graft-versus-leukemia (GvL) and graft-versus-infection (Gvl) effects of a transplant.

The importance and value of transplantation treatment are thus considerably enhanced.

This observation was also surprising and unexpected.

Wings apart-like protein homolog (WAPL) is a protein that in humans is encoded by the WAPL gene (Entrez: 23063). WAPL is a key regulator of the cohesin complex which mediates sister chromatid cohesion, homologous recombination and DNA looping.

Kinesin family member 15 (KIF15) is a protein that in humans is encoded by the KIF15 gene (Entrez: 56992). This gene encodes a motor protein that is part of the kinesin superfamily. KIF15 maintains half spindle separation by opposing forces generated by other motor proteins. KIF15 co-localizes with microtubules and actin filaments in both dividing cells and in postmitotic neurons. Inhibition of KIF15 function has been proposed as a therapeutic approach in cancer chemotherapy.

In still another embodiment of the composition according to the invention the inhibitors are selected from the group consisting of: RNA interference (RNAi) molecule, including small interfering RNA (siRNA), microRNA (miRNA), short hairpin RNA (shRNA), Piwi-interacting RNA (piRNA), and trans-acting siRNA (tasiRNA); antisense oligonucleotide (ASO); small molecule.

Certain regulatory nucleic acids can inhibit gene expression through the biological process of RNA interference (RNAi). RNAi molecules comprise RNA or RNA-like structures typically containing 15-50 base pairs (such as about 18-25 base pairs) and having a nucleobase sequence identical (complementary) or nearly identical (substantially complementary) to a coding sequence in an expressed target gene within the cell.

The length of the RNAi molecules, in particular the siRNA molecules, according to the invention, is typically between about 5 to 30 nucleotides, between about 10 to 30 nucleotides, or about 11, 12, 13, 14, 15, 16, 17, 18, 19, 20, 21, 22, 23, 24, 25, 26, 27, 28, 29, 30 or more nucleotides. siRNA molecules are typically double-stranded RNA. It interferes with the expression of specific genes with complementary nucleotide sequences by degrading mRNA after transcription, preventing translation. The degree of complementarity of the regulatory nucleic acid to the targeted transcript should be at least 75%, at least 80%, at least 85%, at least 90%, or at least 95%.

With this measure, particularly suitable inhibitors are provided in an advantageous way. This is especially true for RNAi molecules, such as siRNA molecules. Because siRNAs are long acting, in particular if chemically modified, they need to be administered much less frequently than prior art therapeutics, such as small molecule inhibitors. As a result, a more balanced therapeutic effect can be expected.

In an embodiment of the invention, the RNAi molecule is virally encoded. This holds true in particular for shRNA molecules.

The composition according to the invention, in an embodiment, can comprise a mixture of siRNA molecules against the above identified targets, i.e., RAN and/or AURKA and/or WAPAL and/or KIF15, but also against other targets. This measure has the advantage that several targets are simultaneously inhibited which may result in further super-additive or synergistic effects against GvHD.

In an embodiment of the invention, the RNAi molecules, such as siRNA molecules, comprise chemically modified nucleotides.

This measure has the advantage that a wide variety of properties can be directionally influenced by targeted chemical modification of the RNAi molecules, including their stability, immunogenicity, toxicity, manufacturability, activity, etc.

In an embodiment of the invention, at least one of the nucleotides of the RNAi molecules comprises a chemical modification or an analogue of a naturally occurring nucleotide. The RNAi or, in particular, siRNA molecule, may include from about 1% to about 100% modified nucleotides or analogues (either in relation to overall nucleotide content, or in relation to one or more types of nucleotide, i.e. any one or more of A, G, U or C) or any intervening percentage (e.g., from 1% to 20%>, from 1% to 25%, from 1% to 50%, from 1% to 60%, from 1% to 70%, from 1% to 80%, from 1% to 90%, from 1% to 95%, from 10% to 20%, from 10% to 25%, from 10% to 50%, from 10% to 60%, from 10% to 70%, from 10% to 80%, from 10% to 90%, from 10% to 95%, from 10% to 100%, from 20% to 25%, from 20% to 50%, from 20% to 60%, from 20% to 70%, from 20% to 80%, from 20% to 90%, from 20% to 95%, from 20% to 100%, from 50% to 60%, from 50% to 70%, from 50% to 80%, from 50% to 90%, from 50% to 95%, from 50% to 100%, from 70% to 80%, from 70% to 90%, from 70% to 95%, from 70% to 100%, from 80% to 90%, from 80% to 95%, from 80% to 100%, from 90% to 95%, from 90% to 100%, and from 95% to 100%).

In an embodiment of the invention the chemical modification is 2'-O-methly and/or 2'fluoro modification.

These chemical modifications of nucleotides have the advantage of providing stability to the RNA and reduce immunogenicity. Other suitable 2' modifications are 2'-O-MOE, LNA (locked nucleic acid), morpholino, (S)-constrained-ethyl, PNA (peptide nucleic acid). Such modifications are, in embodiments, included by the invention.

In yet another embodiment of the invention at least one internucleotide linkage is chemically modified. Preferably, said RNAi molecule comprises nucleotides linked via phosphorothioate.

These chemical modifications of nucleotides have the advantage of giving stability and facilitating cellular uptake as well as reducing immunogenicity of the RNA molecules.

Internucleotide linkage modifications which can improve features of the RNA molecules include methylphosphonate and vinylphosphonate and exNA (extended nucleic acid). An appropriate 5'phosphate modification is vinylphosphonate. All these modifications are encompassed by the invention.

In an embodiment, an analogue of a naturally occurring nucleotide can be, for example, any of pseudouridine (Ψ), N1-methylpseudouridine (me1Ψ), 5-methylcytidine (5mC), phosphorothioate, phosphoramidate, peptide nucleotide, methylphosphonate, 7-deazaguanosine, 2-thiouridine, 5-methyluridine, 5-methyluridine-5'-triphosphate (m5U), 5-iodouridine-5'-triphosphate (15U), 4-thiouridine-5'-triphosphate (S4U), 5-bromouridine-5'-triphosphate (Br5U), 2'-methyl-2'-deoxyuridine-5'-triphosphate (U2'm), 2'-amino-2'-deoxyuridine-5'-triphosphate (U2'NH2), 2'-azido-2'-deoxyuridine-5'-triphosphate (U2'N3), 2'-fluoro-2'-deoxyuridine-5'-triphosphate (U2'F), inosine, 3-methylcytidine, 2-thiocytidine, 2'-methyl-2'-deoxycytidine-5'-triphosphat (C2'm), 2'-amino-2'-deoxycytidin-5'-triphosphate (C2'NH2), 2'-fluoro-2'-deoxycytidine-5'-triphosphate (C2'F), 5-iodocytidin-5'-triphosphate (15U), 5-bromocytidine-5'-triphosphate (Br5C), and 2'-azido-2'-deoxycytidine-5'-triphosphate (C2'N3), 5-azauridine, 2-thio-5-azauridine, 4-thiopseudouridine, 2-thiopseudouridine, 5-hydroxyuridine, 3-methyluridine, 5-carboxymethyl-uridine, 1-carboxymethylpseu-douridine, 5-propynyluridine, 1-propynylpseudouridine, 5-taurinomethyluridine, 1-tauri-nomethylpseudouridine, 5-taurinomethyl-2-thiouridine, 1-taurinomethyl-4-thiouridine, 2-thio-1-methylpseudouridine, 1-methyl-1-deazapseudouridine, 2-thio-1-methyl-1-deazapseudouridine, dihydrouridine, dihydropseudouridine, 2-thiodihydrouridine, 2-thiodi-hydropseudouridine, 2-methoxyuridine, 2-methoxy-4-thiouridine, 4-methoxypseudouridine, 4-methoxy-2-thiopseudouridine, 5-azacytidine, pseudoisocytidine, N4-acetylcytidine, 5-formylcytidine, N4-methylcytidine, 5-hydroxymethylcytidine, 1-methylpseudoisocytidine, pyrrolocytidine, pyrrolopseudoisocytidine, 2-thio-5-methylcytidine, 4-thiopseudoisocytidine, 4-thio-1-methylpseudoisocytidine, 4-thio-1-methyl-1-deazapseudoisocytidine, 1-methyl-1-deazapseudoisocytidine, zebularine, 5-azazebularine, 5-methylzebularine, 5-aza-2-thiozebularine, 2-thiozebularine, 2-methoxycytidine, 2-methoxy-5-methylcytidine, 4-methoxypseudoisocytidine, 4-methoxy-1-methylpseudoisocytidine, 7-deazaadenine, 7-deaza-8-azaadenine, 1-methyladenosine, N6-methyladenosine, N6-isopentenyladeno-sine, N6-(cis-hydroxyisopentenyl)-adenosine, 2-methylthio-N6-(cis-hydroxyisopentenyl)-adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthioadenine, 2-methoxyadenine, 1-methylinosine, 7-deaza-8-azaguanosine, 6-thioguanosine, 6-thio-7-deazaguanosine, 6-thio-7-deaza-8-azaguanosine, 7-methylguano-sine, 6-thio-7-methylguanosine, 7-methylinosine, 6-methoxyguanosine, 1-methylguano-sine, N2-methylguanosine, N2,N2-dimethylguanosine, 8-oxo-guanosine, 7-methyl-8-ox-oguanosine, I-methyl-6-thioguanosine, or N2-methyl-6-thioguanosine or combinations thereof.

In an aspect of this embodiment, the natural occurring nucleotides uridine and/or uridine are replaced by nucleotide analogues, preferably by a nucleotide analogue or a combination of analogues selected from the group consisting of: a combination of pseudouridine for replacement of uridine and 5-methylcytidine for replacement of cytidine, a combination of N1-methylpseudouridine for replacement of uridine and 5-methylcytidine for replacement of cytidine, and N1-methylpseudouridine for replacement of uridine, most preferably N1-methylpseudouridine for replacement of uridine.

In a further embodiment of the invention said RNA interference (RNAi) molecule, preferably siRNA, is targeting RAN and comprises any of the following nucleotide sequences:

| | |
|---|---|
| antisense strand: | SEQ ID NO:1 |
| sense strand: | SEQ ID NO:2 |

In another embodiment of the invention said RNA interference (RNAi) molecule, preferably siRNA, is targeting AURKA and comprises any of the following nucleotide sequences:

| | |
|---|---|
| antisense strand: | SEQ ID NO:3 |
| sense strand: | SEQ ID NO:4 |

Also, in another embodiment of the invention said RNA interference (RNAi) molecule, preferably siRNA, is targeting WAPL and comprises any of the following nucleotide sequences:

| | |
|---|---|
| antisense strand: | SEQ ID NO:5 |
| sense strand: | SEQ ID NO:6 |

Also, in another embodiment of the invention said RNA interference (RNAi) molecule, preferably siRNA, is targeting KIF15 and comprises any of the following nucleotide sequences:

| | |
|---|---|
| antisense strand: | SEQ ID NO:7 |
| sense strand: | SEQ ID NO:8 |

The foregoing embodiments of the invention provide blueprints for such RNAi molecules, in particular siRNA molecules, that have been tested by the inventors and found to be particularly effective in experimental systems.

It goes without saying that the pharmaceutical composition of the invention may comprise a pharmaceutical acceptable carrier. A suitable carrier for the specific treatment will be clear to the skilled person. A suitable carrier for the specific treatment will be clear to the skilled person. Additional disclosure of pharmaceutically acceptable carriers of synthetic siRNA can be found for example in Wang et al. (2010), Delivery of siRNA Therapeutics: Barriers and Carriers, AAPS J. 12(4): 492-503; Karunaratne et al. (2015), Natural Carriers for siRNA Delivery, Curr. Pharm. Des. 21(31):4529-40; serrano-Sevilla et al. (2019), Natural Polysaccharides for siRNA Delivery: Nanocarriers Based on Chitosan, Hyaluronic Acid, and Their Derivatives. The RNA molecules, in particular siRNA molecules, may also be covalently conjugated to cholesterol or other lipids, or to peptides, antibodies or other proteins or to aptamers or to saccharides/sugars or small molecules. Further siRNA delivery methods include packaging into nanoparticles (e.g., polymer-bases nanoparticles), lipid-nanoparticles (LNP), electroporation, viral delivery, etc.

In one embodiment, the pharmaceutical composition is configured for local administration by injection into or external application onto the tissue of the subject, such as a mammal or human, wherein preferably the pharmaceutical composition is present in a formulation or delivery form selected from the group consisting of: a fluid, an implant, a balloon catheter, a vascular prosthesis, a microneedle, a patch, and a stent.

The term "fluid" is to be understood as material continuously deforming when experiencing shear forces. A fluid may have the state of a gas, aerosol, or a liquid.

Any type of external application further is advantageous in that it is less invasive and thus less stressful than other modes of application.

The above-mentioned formulation or delivery form of a fluid is advantageous because fluids allow the application of the pharmaceutical composition by injection, inhalation or the like into virtually any region of the body, no matter the size of the region. In this context the delivery form of a microneedle or a patch allows a low- or non-invasive application, respectively.

In one embodiment, the pharmaceutical composition is configured for systemic administration, wherein preferably the pharmaceutical composition is present in a formulation or delivery form selected from the group consisting of: a fluid or an implant.

The above-mentioned formulation or delivery form of a fluid is advantageous because fluids allow the application of the pharmaceutical composition by injection into virtually any region of the body, no matter the size of the region.

In another embodiment, the RNAi molecules, in particular siRNA molecules, are complexed by polymers or lipids, preferably contained within a lipid nanoparticle, or contained within a liposome. This advantageously further protects the nucleotides from degradation, reduces their immunogenicity, and facilitates its delivery, by improving its entering into the cell.

In the composition according to the invention, in one embodiment, the inhibitors can be the only active ingredients. In another embodiment, additional active agents can be provided.

Another subject-matter of the invention is an inhibitor of RAs-related nuclear protein (RAN) for use in the prophylaxis and/or treatment of graft-versus-host disease (GvHD).

Preferably, other inhibitors are combined with said RAN inhibitor, namely an inhibitor of Aurora kinase A (AURKA) and/or an inhibitor of wings apart-like protein homolog (WAPL) and/or an inhibitor of kinesin family member 15 (KIF15).

The features, characteristics, and advantages mentioned for the composition according to the invention likewise apply to combination of inhibitors according to the invention.

Another subject-matter of the invention relates to a stem cell and a lymphocyte composition, preferably a stem cell and lymphocyte transplant, comprising or having been treated and/or incubated with the composition and/or the inhibitor combination according to the invention, preferably for a period of time allowing the silencing of the above-referenced targets.

With this measure, the transplant or graft is already provided with the invention and can be administered with it, so that a following treatment can be dispensed with. As could been demonstrated by experiments the invention allows silencing of the identified targets for up to 20 or even more cell divisions.

Another subject-matter of the invention relates to a method of producing a pharmaceutical composition for the prophylaxis and/or treatment of graft-versus-host disease (GvHD), comprising the formulation of the inhibitor according to the invention into a pharmaceutically acceptable carrier.

Still another subject-matter of the invention is a method of prophylactical and/or therapeutical treatment of a subject suspected of having or developing graft-versus-host disease (GvHD) or having GvHD, comprising administering the composition and/or the inhibitor and/or the stem cell composition and/or the lymphocyte composition according to the invention to the subject.

The "subject" can be any living being, in particular a mammal, such as a human being.

Other methods that are a subject-matter of the invention involve the ex-vivo-treatment of a stem cell graft with the composition or inhibitor combination according to the invention, and the ex-vivo-treatment of donor lymphocyte preparations with the composition or inhibitor combination according to the invention.

The features, characteristics, and advantages mentioned for the composition according to the invention likewise apply to methods according to the invention.

It is understood that the features mentioned above and those yet to be explained below can be used not only in the particular combination given, but also in other combinations or in isolation, without departing from the scope of the present invention.

The invention is now described and explained in further detail by referring to the following non-limiting examples and drawings.

### BRIEF DESCRIPTION OF THE FIGURES

- Fig. 1:: Screen of siRNAs for silencing and for T cell inhibitory effect.
- Fig. 2:: Screen of siRNA mixes in GvHD model and control T cells.
- Fig. 3:: Screen of siRNA mixes in GvT model.

### EXAMPLES

The inventors selected 7 targets, that were shown to be upregulated the mRNA level in GvHD. It has been unknown, whether these targets drive GvHD or whether they have a mechanistic role at all. Furthermore, it has been unknown, whether these targets play a role in GvL or not.

The inventors designed and screened several siRNA candidates per target gene as shown in the following table:

**Table 1: siRNA seqences. # = phosphorothioate linkage; m = 2'-O-methyl; f = 2'-fluoro; TegChol = cholesterol-triethylene glycol conjugation.**

| Target | Sequence | |
|---|---|---|
| AURKA | antisense | P(mU)#(fU)#(mG)(fU)(fG)(fU)(mA)(fG)(mC)(fG)(mU)(fU)(mC)(fU)#(mA)#(fG)#(mA)#(mU)#(mU)#(mG)#(fA) |
| | sense | (mC)#(mU)#(mA)(fG)(mA)(fA)(mC)(fG)(mC)(fU)(mA)(mC)(mA)(fC)#(mA)#(mA)-TegChol |
| BIM | antisense | P(mU)#(fG)#(mC)(fA)(fA)(fU)(mA)(fC)(mA)(fA)(mA)(fU)(mG)(fC)#(mU)#(fA)#(mC)#(mA)#(mU)#(mA)#(fA) |
| | sense | (mU)#(mA)#(mG)(fC)(mA)(fU)(mU)(fU)(mG)(fU)(mA)(mU)(mU)(fG)#(mC)#(mA)-TegChol |
| KIF15 | antisense | P(mU)#(fC)#(mU)(fU)(fC)(fA)(mU)(fU)(mU)(fA)(mG)(fC)(mU)(fU)#(mU)#(fG)#(mU)#(mU)#(mA)#(mC)#(fA) |
| | sense | (mC)#(mA)#(mA)(fA)(mG)(fC)(mU)(fA)(mA)(fA)(mU)(mG)(mA)(fA)#(mG)#(mA)-TegChol |
| KPNA2 | antisense | P(mU)#(fA)#(mG)(fG)(fU)(fA)(mG)(fG)(mA)(fA)(mG)(fA)(mA)(fU)#(mC)#(fU)#(mG)#(mC)#(mU)#(mC)#(fA) |
| | sense | (mA)#(mG)#(mA)(fU)(mU)(fC)(mU)(fU)(mC)(fC)(mU)(mA)(mC)(fC)#(mU)#(mA)-TegChol |
| RAN | antisense | P(mU)#(fA)#(mG)(fU)(fU)(fG)(mU)(fA)(mG)(fU)(mU)(fA)(mC)(fU)#(mU)#(fU)#(mU)#(mG)#(mG)#(mC)#(fA) |
| | sense | (mA)#(mA)#(mA)(fG)(mU)(fA)(mA)(fC)(mU)(fA)(mC)(mA)(mA)(fC)#(mU)#(mA)-TegChol |
| SYNJ1 | antisense | P(mU)#(fU)#(mG)(fA)(fC)(fC)(mA)(fA)(mG)(fA)(mG)(fG)(mC)(fU)#(mG)#(fC)#(mA)#(mG)#(mA)#(mG)#(fA) |
| | sense | (mG)#(mC)#(mA)(fG)(mC)(fC)(mU)(fC)(mU)(fU)(mG)(mG)(mU)(fC)#(mA)#(mA)-TegChol |
| WAPAL | antisense | P(mU)#(fU)#(mU)(fA)(fG)(fU)(mU)(fA)(mC)(fA)(mU)(fU)(mC)(fU)#(mG)#(fG)#(mU)#(mG)#(mA)#(mG)#(fA) |
| | sense | (mC)#(mC)#(mA)(fG)(mA)(fA)(mU)(fG)(mU)(fA)(mA)(mC)(mU)(fA)#(mA)#(mA)-TegChol |

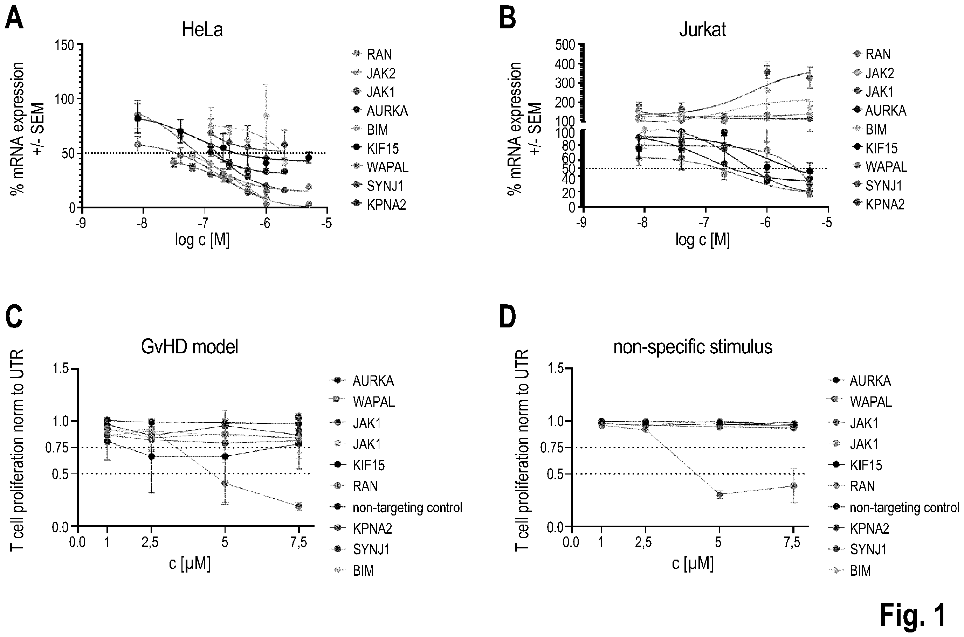

The inventors then performed dose-response studies with lead siRNA candidates to determine their target mRNA inhibitory potency (IC₅₀) into different cell lines: HeLa (Fig. 1A) and Jurkat (Fig. 1B). mRNA expression was measured using QuantiGene SingePlex assay. In case of some siRNAs the inventors observed potent silencing in HeLa but not silencing in Jurkat cells, indicating that mRNA accessibility could be cell-type-dependent. It can be derived from Fig. 1B that RAN siRNA alone already inhibits T cells, i.e. GvHD.

The inventors then tested the potency of siRNAs to inhibit T cell proliferation (Fig. 1 C-D). Primary human T cells were either stimulated with allogeneic irradiated primary human dendritic cells (Fig. 1C, GvHD model), or with CD2/CD3/CD28 beads (Fig. 1D, non-specific stimulus). T cells were labeled with CellTRace Violet fluorescent dye and treated with varying concentrations of siRNAs as indicated. After 5 days of co-culture T cell proliferation was assessed via flow cytometry. The inventors observed potent and dose-dependent T cell proliferation inhibition of RAN siRNA in both models, while other siRNAs did not lead to a significant effect. UTR = untreated. It can be derived from Fig. 1C that RAN siRNA alone already inhibits T cells, i.e. GvHD.

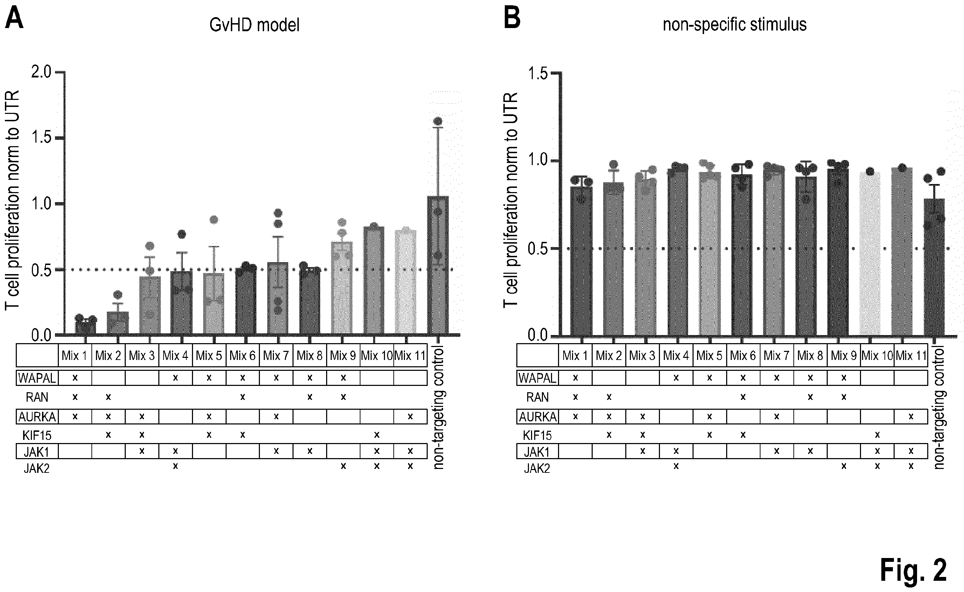

The inventors combined 3 siRNA, 3 µM each, into a mixture and treated primary human T cells either stimulated with allogeneic irradiated T cells (Fig. 2A, GvHD model), or with CD2/CD3/CD28 beads (Fig. 2B, non-specific stimulus). Several siRNA mixes showed a potent T cell inhibition selective to the GvHD situation. Most mixes showed more inhibition, than what could be expected from the addition of the inhibitory effects of the mic components alone. The inventors have not seen T cell toxicity.

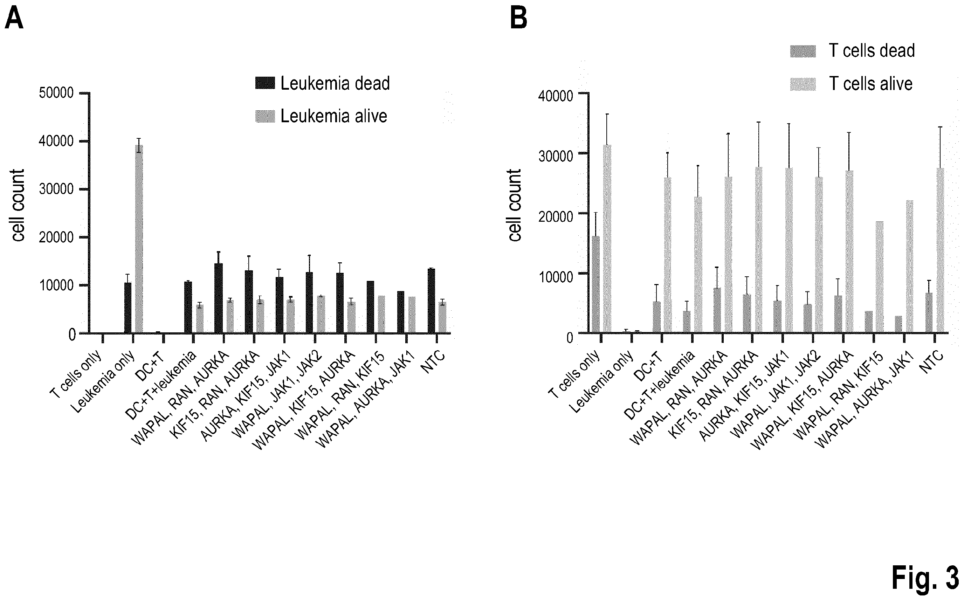

The inventors co-cultured primary human T cells, allogeneic human dendritic cells with the AML leukemia cells line MOLM-13 as a GvT model. They labeled T cells with CellTRace Violet and leukemia cells with CellTrace Red. The inventors then treated this co-culture with various siRNA mixes or non-targeting control (NTC) siRNA for 5 days. They stained for live cells using Zombie and assessed the number of dead and alive leukemia cells (Fig. 3A) and T cells (Fig. 3B). As controls, the inventors cultured T cells, leukemia cells, and T cells together with dendritic cells (GvHD model).

The inventors observed a significant reduction of alive leukemia cells and increase of dead leukemia cells when co-cultured with T cells and dendritic cells (A). The ratio of dead and alive leukemia cells was not altered by siRNA treatment. Furthermore, the number of alive T cells were not significantly altered by siRNA treatment (Fig. 3B). Together, these data suggest the siRNA-treated T cells are still fit to exert leukemia-cytotoxicity.

To summarize, the inventors have designed, screened and identified lead siRNA candidate molecules inhibiting the selected targets and tested them in an *in vitro* GvHD model. siRNAs against RAN showed a potent activity in this assay. Yet, RAN siRNA inhibited T cell proliferation in general, also in a non-specific setting and in a GvT setting. Therefore, the inventors were looking for ways to make the RNA siRNA effect more selective to the GvHD situation. They formed mixes of 2 and 3 siRNAs from the pool of the 7 lead siRNA candidates. The inventors identified before and repeated their functional screening in *in vitro* GvHD, GvT and non-specific setting. Surprisingly they found triple target combinations (WAPAL, AURKA, RAN and AURKA, RAN, KIF15) showing a potent (80-90% inhibition, p=0.0002 and p=0.0006, respectively) inhibition of T cell proliferation upon allogeneic stimulus (GvHD model), yet, showing no inhibition in a non-specific setting and no inhibition in the GvT setting. Treatment with above siRNA mixes did not lead to T cell death. Therefore, above combinations show an unprecedented selectivity for GvHD in *in vitro* models.

### siRNA nucleotide sequences

| SEQ ID | Nucleotide sequence (strand) | | Target |
|---|---|---|---|
| SEQ ID NO:1 | UAGUUGUAGUUACUUUUGGCA | (antisense) | (RAN) |
| SEQ ID NO:2 | AAAGUAACUACAACUA | (sense) | (RAN) |
| SEQ ID NO:3 | UUGUGUAGCGUUCUAGAUUGA | (antisense) | (AURKA) |
| SEQ ID NO:4 | CUAGAACGCUACACAA | (sense) | (AURKA) |
| SEQ ID NO:5 | UUUAGUUACAUUCUGGUGAGA | (antisense) | (WAPAL) |
| SEQ ID NO:6 | CCAGAAUGUAACUAAA | (sense) | (WAPAL) |
| SEQ ID NO:7 | UCUUCAUUUAGCUUUGUUACA | (antisense) | (KIF15) |
| SEQ ID NO:8 | CAAAGCUAAAUGAAGA | (sense) | (KIF15) |
| SEQ ID NO:9 | UGCAAUACAAAUGCUACAUAA | (antisense) | (BIM) |
| SEQ ID NO:10 | UAGCAUUUGUAUUGCA | (sense) | (BIM) |
| SEQ ID NO:11 | UAGGUAGGAAGAAUCUGCUCA | (antisense) | (KPNA2) |
| SEQ ID NO:12 | AGAUUCUUCCUACCUA | (sense) | (KPNA2) |
| SEQ ID NO:13 | UUGACCAAGAGGCUGCAGAGA | (antisense) | (SYNJ1) |
| SEQ ID NO:14 | GCAGCCUCUUGGUCAA | (sense) | (SYNJ1) |

## Claims

1. A pharmaceutical composition for the prophylaxis and/or treatment of graft-versus-host disease (GvHD) comprising an inhibitor of RAs-related nuclear protein (RAN).

2. The pharmaceutical composition of claim 1, additionally comprising an inhibitor of Aurora kinase A (AURKA).

3. The pharmaceutical composition of claim 1 or 2, additionally comprising an inhibitor of wings apart-like protein homolog (WAPL).

4. The pharmaceutical composition of any of the preceding claims, additionally comprising an inhibitor of kinesin family member 15 (KIF15).

5. The pharmaceutical composition of any of the preceding claims, wherein the inhibitors are selected from the group consisting of: RNA interference (RNAi) molecule, including small interfering RNA (siRNA), microRNA (miRNA), short hairpin RNA (shRNA), a Piwi-interacting RNA (piRNA), and trans-acting siRNA (tasiRNA); antisense oligonucleotide (ASO); small molecule.

6. The pharmaceutical composition of claim 5, wherein said RNA interference (RNAi) molecule, preferably siRNA, is targeting a target selected from the group consisting of: RAN, AURKA, WAPL, and KIF15.

7. The pharmaceutical composition of claim 5 or 6, wherein said RNAi molecule comprises chemically modified nucleotides.

8. The pharmaceutical composition of claim 7, wherein the chemical modification is 2'-O-methly and/or 2'fluoro.

9. The pharmaceutical composition of any of claims 5-8, wherein RNAi molecule comprises nucleotides linked via phosphorothioate.

10. An inhibitor of RAs-related nuclear protein (RAN) for use in the prophylaxis and/or treatment of graft-versus-host disease (GvHD).

11. The inhibitor of claim 10, additionally comprising an inhibitor of Aurora kinase A (AURKA) and/or an inhibitor of wings apart-like protein homolog (WAPL) and/or an inhibitor of kinesin family member 15 (KIF15).

12. A stem cell composition, preferably a stem cell transplant, comprising the composition of any of claims 1-9 and/or the inhibitor of claim 10 or 11.

13. A lymphocyte composition, preferably a lymphocyte transplant, comprising the composition of any of claims 1-9 and/or the inhibitor of claim 10 or 11.

14. A method of producing a pharmaceutical composition for the prophylaxis and/or treatment of graft-versus-host disease (GvHD), comprising the formulation of the inhibitor of claim 10 or 11 into a pharmaceutically acceptable carrier.

15. A method of prophylactical and/or therapeutical treatment of a subject suspected of having or developing graft-versus-host disease (GvHD) or having GvHD, comprising administering the pharmaceutical composition of any of claims 1-9 and/or the inhibitor of claim 10 or 11 and/or the stem cell composition of claim 12 and/or the lymphocyte composition of claim 13 to the subject.
